# EUROPEAN PATENT APPLICATION

(11) **EP 4 647 069 A1**
(43) Date of publication of application: **12.11.2025**
(21) Application number: 25174732.5
(22) Date of filing: 07.05.2025
(51) Int. Cl.: A61K 31/12, A61K 31/4015, A61K 31/57, A61K 31/593, A61K 31/7072, A61P 25/00

(54) **DRUGS AND COMPOSITIONS FOR USE IN THE TREATMENT OF RETT SYNDROME**

(30) Priority: 07.05.2024 IT 202400010198
(71) Applicant: UNIVERSITA' DEGLI STUDI DI TRIESTE, 34127 Trieste (IT)
(72) Inventor: Tongiorgi, Enrico, 34123 Trieste (TS) (IT); Roggero, Ottavia Maria, 07100 Sassari (SS) (IT); Berutto, Vittoria, 10025 Pino Torinese (TO) (IT)
(74) Representative: Petraz, Gilberto Luigi

(57) **Abstract**

The present invention concerns a composition for use for, or for the treatment of, Rett syndrome, which proposes a repositioning of a selective and reversible catechol-O-methyltransferase (COMT) inhibitor.

## Description

### FIELD OF THE INVENTION

The present invention concerns drugs and compositions for use in the treatment of Rett syndrome.

### BACKGROUND OF THE INVENTION

Rett syndrome (RTT) is a rare genetic disorder that affects the development of the nervous system and affects predominantly female individuals. RTT represents one of the most common causes of mental retardation in females and affects 1:10,000 newborns worldwide (Chahrour and Zoghbi, 2007; Williamson and Christodoulou, 2006). In 95% of cases, RTT is caused by mutations linked to the X chromosome, in the gene encoding the methyl CpG binding protein 2 (MECP2) (Amir et al. 1999; Dragich et al. 2000). Most of these consist of de novo mutations, with over 200 single nucleotide changes identified, but can also be caused by frameshifts, deletions, nonsense and missense mutations (Renieri et al. 2003; Williamson and Christodoulou 2006).

A feature common to all RTT patients is the deceleration of brain growth leading to microcephaly. The arrest of brain growth is associated in RTT with neuronal atrophy (Belichenko et al., 1994; Kaufmann et al., 2000; Fukuda et al., 2005). Neuronal atrophy, defined as a reduced extension of the cellular processes of neurons (axons and dendrites) and their branches, accompanied by a small cell body is a salient feature of many neurological diseases. In RTT patients, brain atrophy is visible from MRI scans at the level of the cerebral cortex, particularly the motor cortex, basal ganglia, thalamus and hippocampus, and in the cerebellum (Armstrong et al., 1995, Bauman et al., 1995; Armstrong, 2002; Belichenko et al., 2008; Pohodich and Zoghbi, 2015). In addition, post-mortem histological analysis of RTT brains has revealed, in the atrophied brain regions, the presence of smaller and denser than normal neurons with a reduced dendritic complexity in the frontal lobes, hypothalamus, and hippocampus. These abnormalities are an important hallmark of RTT, and it has been hypothesized that the decrease in dendritic complexity, especially at the cortex level, is the leading cause of neural network failure (Bauman et al., 1995). Indeed, the reduction of the area of the neuronal soma and dendritic arborization have been associated with intellectual disability, epilepsy, and dysfunctions of specific brain regions that cause the distinctive symptoms of RTT (Weng et al., 2011).

Demonstrating that the reactivation of the *MECP2* gene in a mouse model without MeCP2 is able to recover much of the phenotype in this animal model of Rett syndrome, including cortical atrophy, has highlighted that the disease is reversible, paving the way for the search for new treatments (Guy, 2007). In particular, the reactivation of the *MeCP2* gene in MeCP2-KO mice showed an almost complete reversal of the disease and an increase in survival (Guy et al., 2007).

In other studies, the overexpression of the Brain-derived neurotrophic factor (BDNF) neurotrophin in MeCP2-KO mice extended their lifespan, resolving some locomotor symptoms and electrophysiological defects (Chang et al., 2006). However, even if symptoms disappear when gene expression of *MeCP2* is restored in MeCP2-KO mice, gene therapy for RTT may not be accessible or appropriate for all patients affected by Rett syndrome.

There is therefore a high interest in the development of drug treatments effective for RTT.

Several tests have been done with several drug candidates that have different mechanisms of action from each other. These tests have allowed to achieve positive but not entirely satisfactory results.

The results achieved so far suggest that pharmacological treatment with effective compounds may lead to significant improvements in the RTT phenotype.

US-A1-2015/104506 concerns compositions able to be used in the treatment of movement disorders. Among the various combination therapy options, tolcapone is indicated as a possible active ingredient. Disorders associated with Parkinson disease, including Rett syndrome, are cited among the various movement disorders. However, treatment of Rett syndrome with tolcapone is not directly and unequivocally disclosed.

Panayotis Nicolas et al. "State-of-the-art therapies for Rett syndrome", Developmental Medicine & Child Neurology, Vol. 65, no. 2, p. 162-170 (XP093216620) describes the state of the art of therapies for Rett syndrome and is silent about tolcapone as a potential therapeutic agent.

Chapleau Christopher et al. "Evaluation of Current Pharmacological Treatment Options in the Management of Rett Syndrome: From the present to Future Therapeutic Alternatives", Current Clinical Pharmacology, Vol. 8, no. 4, p. 358-369 (XP093071740) concerns drug treatment options in the management of Rett syndrome, but does not mention tolcapone and its potential therapeutic role in the management of Rett syndrome.

There is therefore the need to perfect a composition for use in the treatment of Rett syndrome that can overcome at least one of the disadvantages of the state of the art.

To do this, it is necessary to resolve the technical problem of proposing a composition for use in the treatment of Rett syndrome that has an alternative mechanism of action to known compositions.

In particular, one purpose of the present invention is to provide a composition for use for Rett syndrome that allows to act directly on neurons in order to improve the disease condition thereof, which various symptoms are associated with, including neuronal atrophy.

The Applicant has devised, tested and embodied the present invention to overcome the shortcomings of the state of the art and to obtain these and other purposes and advantages.

### SUMMARY OF THE INVENTION

The present invention is set forth and characterized in the independent claims. The dependent claims describe other characteristics of the present invention or variants to the main inventive idea.

In accordance with the above purpose and to resolve the technical problem described above in a new and original way, also achieving considerable advantages compared to the state of the prior art, the present invention concerns tolcapone for use in the treatment of Rett syndrome.

According to another aspect, the invention concerns a composition for use in the treatment of Rett syndrome that comprises the drug tolcapone as active ingredient.

This use represents a case of repositioning the drug with respect to its intended use known to date. Tolcapone is a nitrocatechol with formula C₁₄H₁₁NO₅ and it is orally active. Its chemical structure is as follows:

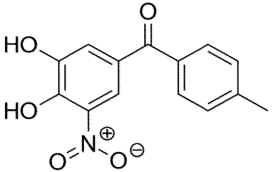

Tolcapone is a drug classified as a selective and reversible inhibitor of the enzyme catechol-O-methyltransferase (COMT), one of the enzyme systems that metabolize levodopa. It is currently approved only for the treatment of Parkinson's disease and is used in combination with levodopa/benserazide or levodopa/carbidopa in patients with levodopa-responsive idiopathic Parkinson's disease and motor fluctuations, who have not responded to or who cannot tolerate other COMT inhibitors.

Other catechol-O-methyltransferase (COMT) inhibitor drugs are known, which can be considered as alternatives to tolcapone, in particular entacapone, nebicapone, nitecapone and opicapone.

Tolcapone is a pharmaceutical ingredient that, when applied in Rett syndrome, is capable of reactivating the maturation process of neurons that present atrophy because they are blocked at an immature stage of their development, and therefore the dendrites have a reduced extension, and a smaller number of branches and synapses compared to normal neuronal development.

Tolcapone, unlike its analogue most clinically used in Parkinson's disease - entacapone, has shown to have excellent capabilities to penetrate the blood-brain barrier and to have a longer half-life (2.9 h compared to 0.8 h), and it is therefore the drug of this class most suited to have a direct effect on the brain (Forsberg et al., 2003).

The effect of tolcapone as an agent capable of promoting neuronal maturation has never been described before in any other pathology of the nervous system, and was not predictable based on the mechanism of action as a COMT inhibitor, which is prevalent in the current scientific literature.

Through a bioinformatics analysis aimed at identifying possible new and specific mechanisms of action for tolcapone, all human proteins with pockets were analyzed, for a total of 25,717 proteins, to which tolcapone can bind. From this analysis, the aldo-keto reductase family 1, member b1 (AKR1B1, alias ALDR1) was identified as the protein with the highest probability of binding to tolcapone. AKR1B1 was therefore found to be in 1^{st} place in a ranking based on the highest binding probability, while the COMT protein, considered as the main target for the drug's action in Parkinson's disease, was found to be in 26^{th} place in a ranking based on the highest binding probability.

AKR1B1 is an enzyme involved in various functions, including prostaglandin synthesis. Under normal conditions, phospholipid is transformed into arachidonic acid in a reaction catalyzed by the enzyme phospholipase A2 (PLA2G). Subsequently, arachidonic acid is converted into prostaglandin H2 (PGH2) with the help of cyclooxygenase 1 (COX1) and cyclooxygenase 2 (COX2). AKR1B1 consumes NADPH and converts PGH2 into prostaglandin F2alpha (PGF2A). It has been proposed that increased amounts of reactive oxidant species (ROS) may lead to the activation of a lipid peroxidation process involving arachidonic acid. When arachidonic acid is attacked by an ROS, the double bond structure of arachidonic acid undergoes a complex series of radical rearrangements, which end in metabolites with three OH groups. These metabolites, called F2-isoprostanes, are mediators of oxidative stress in vivo and have been implicated as part of the mechanism leading to the increased oxidative damage in Rett syndrome (Signorini et al., 2013; De Felice et al., 2014).

Therefore, an innovative aspect of the present invention is to have identified a possible new mechanism of action of tolcapone that would function as an antiinflammatory agent, in particular for the arachidonic acid signaling cascade. This occurs because the formation of PGF2A mediated by the enzyme AKR1B1, a target protein of tolcapone, is in antithesis to the formation of F2-isoprostanes stimulated by the ROS and implicated in the increased oxidative stress typical of Rett syndrome.

In accordance with another aspect of the present invention, the composition comprises tolcapone combined with another drug chosen from calcipotriene, idoxuridine, melengestrol acetate and rolipram. Among these drugs, calcipotriene and idoxuridine are the most effective, since they allow, when combined with tolcapone, to obtain good results at lower concentrations than the usual concentrations of drugs of the state of the art. The present invention also concerns the combination of tolcapone and at least one other drug chosen from calcipotriene, idoxuridine, melengestrol acetate and rolipram for the treatment of Rett syndrome.

Advantageously, when tolcapone is combined with one of these drugs, the concentration ratio tolcapone:other drug is comprised between 1:0.1 and 1:10, preferably between 1:0.1 and 1:1, more preferably it is equal to 1:1.

In one variant, the composition comprises the drugs tolcapone and calcipotriene. In this variant, both drugs can have a concentration comprised between 0.2 µM and 5 µM, preferably between 0.5 µM and 2 µM, more preferably between 0.8 µM and 1.5 µM. A possible concentration of these two drugs can be, for example, 1 µM.

In another variant, the composition comprises the drugs tolcapone and idoxuridine. In this variant, both drugs can have a concentration comprised between 0.02 M and 0.5 µM, preferably between 0.05 µM and 0.2 µM, more preferably between 0.08 µM and 0.15 µM. An example of a concentration of these two drugs can be 0.1 µM.

### DESCRIPTION OF THE DRAWINGS

These and other aspects, characteristics and advantages of the present invention will become apparent from the following description of an embodiment, given as a non-restrictive example with reference to the attached drawings wherein:
- fig. 1 shows the developmental stages of hippocampal neurons in culture and the times at which cells enter each developmental stage (Dotti, Sullivan and Banker 1988);
- figs. 2A-2D show the morphological characterization of hippocampal neurons from WT (normal) and MeCP2-KO mice;
- figs. 3A-3E show the results of a primary screening of 640 drugs performed on the 6^{th} day in culture (DIV 6, days in vitro 6);
- figs. 4A-4B show dose-response curves, in re-screening, of tolcapone at DIV 6;
- figs. 5A-5D are test results showing the effect of tolcapone and calcipotriene taken individually, compared with the effect of these two drugs as a pair;
- figs. 6A-6D are test results showing the effect of tolcapone and idoxuridine taken individually, compared to the effect of these two drugs as a pair;
- fig. 7 shows a schematized neuron with the branches numbered in progression from the outermost to the closest to the neuron's cell body;
- figs. 8A-8B summarize the test results showing the quantification of the recovery for the five morphological parameters in MeCP2-KO neurons at DIV 12 following treatment with tolcapone.

We must clarify that the phraseology and terminology used in the present description, as well as the figures in the attached drawings also in relation as to how described, have the sole function of better illustrating and explaining the present invention, their purpose being to provide a non-limiting example of the invention itself, since the scope of protection is defined by the claims.

To facilitate comprehension, the same reference numbers have been used, where possible, to identify identical common elements in the drawings. It is understood that elements and characteristics of one embodiment can be conveniently combined or incorporated into other embodiments without further clarifications.

### DESCRIPTION OF SOME EMBODIMENTS

Unless otherwise defined, all the technical and scientific terms used here and hereafter have the same meaning as commonly understood by a person with ordinary experience in the field of the art to which the present invention belongs. Even if methods and materials similar or equivalent to those described here can be used in practice and in the trials of the present invention, the methods and materials are described hereafter as an example. In the event of conflict, the present application shall prevail, including its definitions. The materials, methods and examples have a purely illustrative purpose and shall not be understood restrictively.

All measurements are carried out at 25°C (ambient temperature) and at atmospheric pressure, unless otherwise indicated. All temperatures are in degrees Celsius, unless otherwise indicated.

All percentages and ratios indicated shall be understood to refer to the weight of the total composition (w/w), unless otherwise indicated.

All percentage ranges indicated here are given with the provision that the sum with respect the overall composition is 100%, unless otherwise indicated.

All the intervals reported here shall be understood to include the extremes, including those that report an interval "between" two values, unless otherwise indicated.

The present description also includes the intervals that derive from uniting or overlapping two or more intervals described, unless otherwise indicated.

The present description also includes the intervals that can derive from the combination of two or more values taken at different points, unless otherwise indicated.

Some aspects described here concern methods and uses of tolcapone for the treatment of Rett syndrome, optionally in combination with at least one other drug chosen from calcipotriene, idoxuridine, melengestrol acetate and rolipram for the treatment of Rett syndrome.

Some embodiments described here also concern compositions comprising tolcapone and possibly one or more of either calcipotriene, idoxuridine, melengestrol acetate and rolipram for the treatment of Rett syndrome.

The compositions described here, therefore, can be pharmaceutical compositions. The term "pharmaceutical composition" as used here is intended to comprise a composition suitable for administration to a subject, such as a mammal, especially a human. In general, a "pharmaceutical composition" is preferably sterile and free of contaminants capable of eliciting an undesired response in the subject (for example, the compound(s) in the pharmaceutical composition is/are pharmaceutical grade).

The compositions described here, therefore, can include therapeutically effective amounts of tolcapone and possibly therapeutically effective amounts of one or more of either calcipotriene, idoxuridine, melengestrol acetate, and rolipram.

The expression "therapeutically effective amount" as used here indicates the amount of a compound that, when administered to a mammal or other subject for the treatment of a disease, is sufficient to perform such treatment for the disease. The "therapeutically effective amount" will vary depending on the compound, the disease, its severity and the subject's age, weight, etc.

In some embodiments, compositions described here can include a pharmaceutical carrier.

In one embodiment, one or more of the drugs described here can be conjugated to the at least one pharmaceutical carrier. In another embodiment, one or more of the drugs described here can be encapsulated in, or associated with, the at least one pharmaceutical or pharmaceutically acceptable carrier.

In a particular embodiment, the at least one pharmaceutical carrier is chosen from the group consisting of carbohydrates, lipids, peptides, proteins, nucleic acid molecules, synthetic polymers or combinations thereof.

In one embodiment, the pharmaceutical carrier is a nanotransporter. In a particular embodiment, the nanotransporter comprises one or more of either cationic lipids, cationic polymers, cationic peptides, or combinations thereof.

The expression "pharmaceutically acceptable carrier" as used in the present description refers to a medium, composition or formulation that allows for an effective delivery of the therapeutic agent to the physical location most suited to the desired activity. It includes pharmaceutically acceptable materials, compositions or carriers, such as a liquid or solid filler, diluent, solvent or encapsulating material involved in carrying or conveying any composition in question from one organ, or part of the body, to another organ, or part of the body. Each carrier has to be "acceptable", in the sense that it is compatible with the other ingredients of a composition in question and not harmful to the patient.

The compositions suitable for the purposes described here can be liquid, solid, semi-solid, powder.

The compositions described here can be packaged as such or mixed with acceptable excipients and conveniently formulated in any form suitable for parenteral, enteral or oral administration, including solid forms such as powders, granules, sachets, stick-packs, flow-packs, capsules, possibly normal or controlled release tablets, for example time or pH dependent, or film-coated, or gastro-protected, colon-specific or multilayer release, chewing gum or candies, or liquid forms such as syrups, drops, elixirs, solutions and suspensions in general.

In addition, the compositions described here can be incorporated into other formulations, such as an aqueous solution for administration by intranasal spray and suchlike, suppositories, ovules, transdermal patches and suchlike.

For example, the compositions described here can be mixed with acceptable excipients and conveniently formulated to be employed in any form whatsoever suitable for pharmaceutical use.

Tolcapone and possibly one or more of the other drugs described here can be administered alone or together with a pharmaceutical carrier, selected in relation to the intended form of administration and in accordance with conventional methods.

The pharmaceutical compositions can be specifically formulated for administration by any suitable route such as oral, rectal, nasal, topical (including buccal and sublingual), transdermal and parenteral (including subcutaneous, intramuscular, intrathecal, intravenous and intradermal). The therapeutic agents can be administered in liquid or solid form.

In one embodiment, one or both of the therapeutic agents (that is, tolcapone and possibly one or more of the other drugs described here) can be administered intravenously or intraperitoneally by infusion or injection. Solutions of the therapeutic agent or its salts can be prepared in water or saline solution, possibly mixed with a non-toxic surfactant. Dispersions can be prepared in glycerol, liquid polyethylene glycols, triacetin and mixtures thereof, and in oils. Under normal conditions of storage and use, these preparations contain a preservative to prevent the growth of microorganisms. Pharmaceutical dosage forms suitable for injection or infusion can include sterile aqueous solutions or dispersions, or sterile powders, comprising the active ingredient, which are suitable for the extemporaneous preparation of sterile injectable or infusible solutions or dispersions, optionally encapsulated in liposomes. The final dosage form is optionally sterile, fluid and stable under the conditions of production and storage. The carrier or liquid carrier can be a solvent or liquid dispersion medium comprising, for example, water, ethanol, a polyol (for example glycerol, propylene glycol, liquid polyethylene glycols and suchlike), vegetable oils, non-toxic glyceryl esters and suitable mixtures thereof. In one embodiment, one or both of the therapeutic agents can be combined with one or more excipients and used in the form of ingestible tablets, orosoluble tablets, troches, capsules, elixirs, suspensions, syrups, wafers and suchlike. These compositions and preparations can contain at least 0.1% (w/w) of at least one therapeutic agent. The percentage of the compositions and preparations may of course be varied, for example from about 0.1% to almost 100% of the weight of a given unit dosage form. The amount of the at least one therapeutic agent is such that an effective dose level will be achieved at the time of administration.

The tablets, lozenges, pills, capsules and suchlike can also contain one or more of the following: binders, such as microcrystalline cellulose, gum tragacanth, acacia, corn starch or gelatin; excipients, such as dicalcium phosphate, starch or lactose; a disintegrating agent, such as corn starch, potato starch, alginic acid, primojel and suchlike; a lubricant, such as magnesium stearate; a glidant, such as colloidal silicon dioxide; a sweetening agent, such as sucrose, fructose, lactose, saccharin or aspartame; a flavoring agent, such as peppermint, methylsalicylate, wintergreen oil or cherry flavoring; a peptide antibacterial agent; and an excipient, such as croscarmellose sodium, povidone, hypromellose, titanium dioxide. When the unit dosage form is a capsule, it can contain, in addition to the above-mentioned materials, a liquid carrier, such as a vegetable oil or polyethylene glycol. Various other materials can be present as coatings, or to otherwise modify the physical shape of the solid unit dosage form. In one embodiment, one or more of the therapeutic agents are prepared with carriers that will protect the compound from rapid elimination from the body, such as a controlled release formulation, including implants and microencapsulated delivery systems. Biodegradable and biocompatible polymers, such as modified conjugated cellulose, ethylene vinyl acetate, polyanhydrides, polyglycolic acid, collagen, polyorthoesters and polyacetic acid can be used. In one embodiment, tolcapone and possibly one or more of the other drugs described here are co-administered at a systemic level. In one particular embodiment, tolcapone and possibly one or more of the other drugs described here is administered orally, while in another particular embodiment it is administered by means of intravenous or intraperitoneal administration.

Administration can be done by means of any conventional method or route for the administration of therapeutic agents, including systemic or localized routes. In general, contemplated routes of administration include, but are not necessarily limited to, intranasal, intrapulmonary, intramuscular, intratracheal, subcutaneous, intradermal, topical, intravenous, rectal, nasal, oral routes, and other parenteral routes of administration. Routes of administration can be combined, if desired, or adjusted according to the agent and/or effect desired.

A person of skill in the art will easily appreciate that dose levels may vary depending on the specific agent, severity of symptoms, and the subject's susceptibility to any side effects.

Although the dosage used will vary depending on the clinical goal to be achieved, in one embodiment, tolcapone and possibly one or more of the other drugs described here is administered in a fixed dose.

Depending on the dosage, it may be possible or convenient to administer the daily dose in multiple dosage units.

In one embodiment, administration occurs once a day. In another embodiment, administration occurs twice a day. In yet another embodiment, administration occurs three, four, five, or six times per day. In one embodiment, administration occurs once every other day. In another embodiment, administration occurs once a week, a month or year. In another embodiment, administration occurs two, three, or four times or more per week, per month, or per year.

Administration time may vary. In one embodiment, administration occurs in the morning, mid-morning, noon, afternoon, evening or midnight.

### EXPERIMENTAL DATA

Given the importance of neuronal atrophy as a causative event of a series of abnormalities in the functioning of the nervous system and the potential for recovery of the symptoms of Rett syndrome, an in vitro phenotypic screening of drugs was undertaken using the "neuronal atrophy" phenotype found in cultured MeCP2-KO mouse neurons, used due to being an internationally validated in vitro model of Rett syndrome (Guy et al., 2001; Katz et al., 2012).

As a first point to be able to carry out the drug screening on MeCP2-KO mouse neurons, the known fact that neurons extracted from the rat hippocampus and cultured under appropriate conditions reach mature neuronal morphology over several days in vitro (DIV) was considered. In 1988, Dotti and collaborators identified five main stages of this in vitro development process and defined the day (DIV) when the neurons enter each stage.

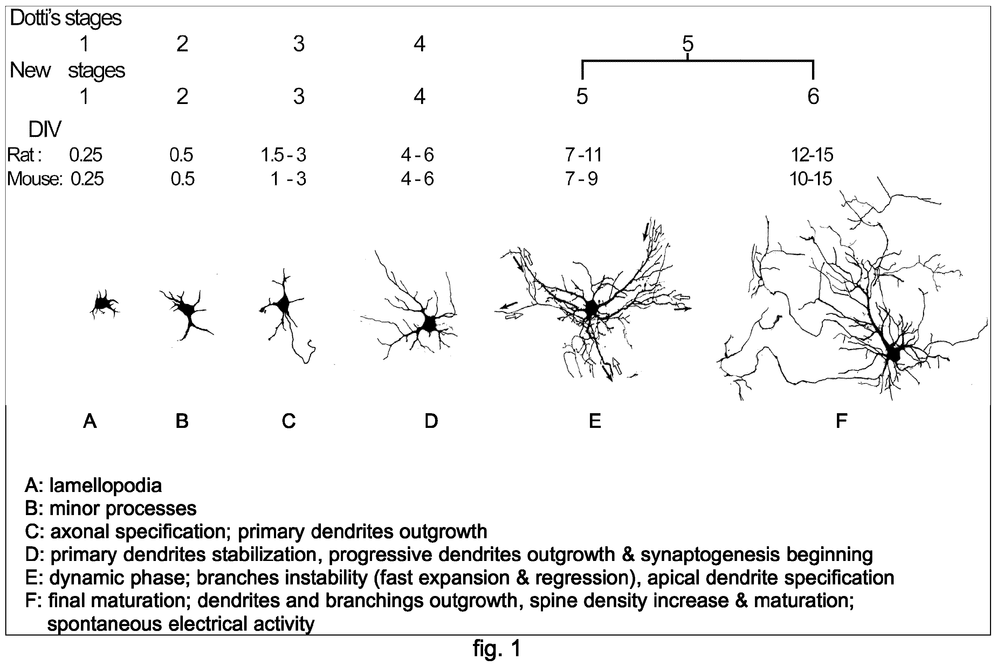

However, in order to conduct an in vitro pharmacological screening on MeCP2-KO mouse neurons, it was necessary to collect data on the in vitro development of mouse neurons. The comparative analysis of the in vitro developmental stages of rat and mouse hippocampal neurons allowed to review the stages defined in 1988 by Dotti and colleagues (fig. 1, taken from Baj et al., 2014). Based on the updated data, stage 3 previously described by Dotti et al. as restricted to DIV 2 was extended from DIV 1.5 to DIV 3. Stage 4 was repositioned as included between DIV 4 and DIV 6, and it was found that at this stage the primary dendrites were stabilized, and synaptogenesis was already beginning.

In addition, stage 5 was redefined from DIV 7 to DIV 9, as the stage during which the dendritic branches become unstable presenting rapid phases of expansion and regression that culminate in the specification of the apical dendrite and in the maturation of the dendritic spines. In addition, at this stage the synaptogenesis is almost complete.

Finally, a new stage 6 was added, comprising DIV 10-15, in which the apical dendrite becomes mature and a general outgrowth of the dendrites and an increase in the density of the dendritic spines is observed (fig. 1).

The in vitro model of mouse hippocampal neuron development described above was then used to identify the exact stage at which the structural failure of neurons occurs in Rett syndrome. For this purpose, hippocampal neurons cultured from MeCP2-KO mice were used to evaluate their total dendritic length, the number of primary and secondary dendrites and the branch points at DIV 6, 9 and 12, which were defined to be the critical points in the transition of the development to mature neurons.

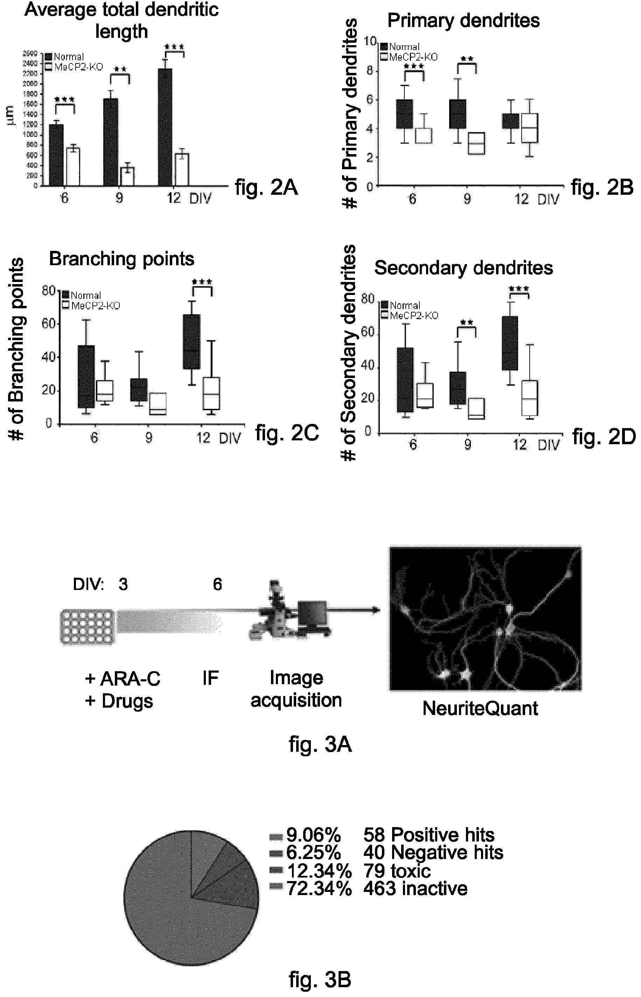

While in controls with normal genotype (WT) the total dendritic length progressively increases at DIV 6, 9 and 12, quantitative analysis revealed in MeCP2-KO neurons a lower degree of dendritic extension already at DIV 6 (total mean length at DIV 6: WT: 1206±73.8 µm; KO: 742.7±73 µm) and this length does not increase over time (fig. 2A, taken from Baj et al., 2014). In addition, the MeCP2-KO neurons have a lower number of primary dendrites at DIV 6 than WT neurons, to then recover in the following phases reaching a mean of 4 primary dendrites as in WT neurons (fig. 2B). Finally, with regard to the growth of new dendritic branches, a mean number of branch points and secondary dendrites was found, the same for WT and MeCP2-KO neurons at DIV 6. However, in the subsequent phases of neuronal development (DIV 9 and DIV 12), while the WT neurons grow considerably, in the MeCP2-KO neurons the number of branch points and the number of secondary dendrites remains comparable to that observed at DIV 6 (fig. 2C and 2D).

In a following phase, miniaturized cultures of MeCP2-KO mouse hippocampal neurons that retain the same characteristics as neuron cultures used in previous studies were developed, and three morphological biomarkers were identified, useful for significantly detecting dendrite atrophy compared to genetically normal (WT) neurons (Nerli et al., 2020).

Specifically, while the initial study (Baj et al., 2014) was conducted on cultures in 24-well plates, in this phase the culture conditions of the primary hippocampal neurons were optimized to obtain a miniaturized, reproducible and robust cellular assay in 96-well plates (Nerli et al., 2020).

After analyzing the impact of the different culture conditions on the in vitro morphological and functional maturation of primary cultures of hippocampal neurons of WT mice, a culture protocol in 96-well plates at cell densities ranging from 320 to 160 cells/mm² was established.

This protocol has been shown to preserve normal development and a functional neuronal network, and it allows to carry out a phenotypic screening of drugs for RTT dendritic atrophy. Methodologically, neurons fixed and labeled for immunofluorescence with antibodies against the protein Microtubule associated protein 2 (MAP2), a biomarker for dendritic processes, and the protein NeuN, a biomarker for the neuronal cell body, were observed under a fluorescence microscope and using high-content imaging techniques with the software NeuriteQuant.

Three morphological parameters were measured: 1) the area of the neuronal cell body (soma), 2) the total dendritic length (TDL), 3) the number of dendritic endpoints (EP). As a pilot test to demonstrate the efficacy of this cellular assay in identifying compounds capable of counteracting dendritic atrophy, the miniaturized cultures of MeCP2-KO mouse neurons were incubated for 3 days, from DIV 9 to DIV 12, with the Brain-derived neurotrophic factors (BDNF) neurotrophin and the antidepressant mirtazapine, and the effects were analyzed with NeuriteQuant at DIV 12. The results published in Nerli et al., 2020 demonstrate that this cellular assay is suitable for the identification of treatments that counteract neuronal atrophy.

In a fourth phase, the phenotypic screening assay developed in Nerli et al. 2020, was adapted into a configuration wherein the miniaturized cultures were treated with the drugs for 3 days using more immature cultures than those used by Nerli et al., 2020, that is, using a time window from DIV 3 to DIV 6 for the treatment. The in vitro model used for the primary screening is shown in fig. 3A.

In a fifth phase, the primary screening of the ENZO Screen Well V1 library containing 640 FDA-approved drugs was conducted, using the assay modified by Nerli et al., 2020 adapted to the cultures of immature hippocampal neurons (fig. 3A). In particular, from the Venn diagram shown, a total of 640 drugs tested can be seen, 58 ("*positive feedback*") had a positive effect on the TDL and/or EP, while 40 drugs ("*negative feedback*") had a negative effect on the TDL and/or EP, 79 drugs ("toxic") had a toxic effect, and 463 drugs ("inactive") had neither a positive nor negative effect on the TDL and/or EP (fig. 3B).

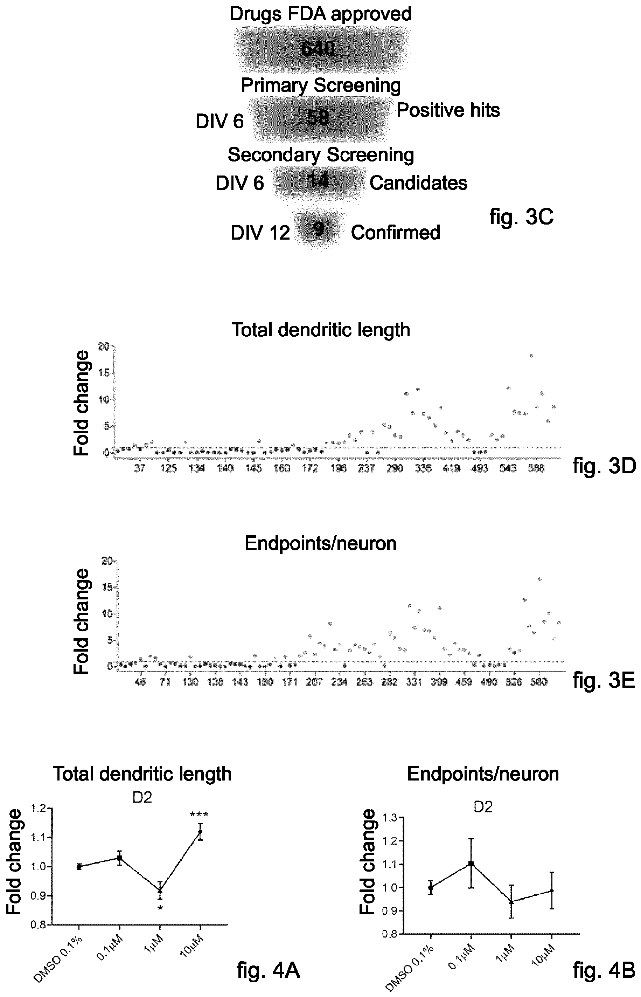

Fig. 3C graphically shows the screening starting from 640 drugs up to the confirmation of 14 drugs at DIV 6, and 9 drugs at DIV 12.

From the primary screening of the 640 compounds of the ENZO Screen Well V1 library, conducted at a concentration of 10 µM on neurons treated from DIV 3 to DIV 6, 58 compounds were identified capable of promoting an increase in total dendritic length (TDL) and/or dendritic endpoints (EP) (light grey dots; figs. 3D and 3E), and 40 drugs were identified that worsen these two parameters (dark grey dots; figs. 3D and 3E), with respect to the same cultures treated with the carrier substance consisting of Dimethylsulfoxide (DMSO) at a concentration of 0.1% in water.

In detail, 42 drugs (light grey dots) had a positive effect on the TDL, 36 drugs (dark grey dots) had a negative effect on the TDL (fig. 3D). Regarding the dendritic endpoints, 52 drugs (light grey dots) had a positive effect on EP, 37 drugs (dark grey dots) had a negative effect on EP (fig. 3E). An n=1 independent experiment (one cell culture only) was performed. The Kruskall-Wallis test was performed to compare non-parametric data between more than two groups: *p <0.05, **p<0.01, ***p<0.001.

In a sixth phase, the 58 positive compounds were re-tested through incubation for 3 days from DIV 3 to DIV 6, at concentrations of 0.1 µM, 1 µM and 10 µM. 14 drugs were confirmed following this re-screening. The drug tolcapone (D2) figures among these 14 drugs capable of counteracting neuronal atrophy in MeCP2-KO mouse neurons, tolcapone (D2) being found to be effective in making the total dendritic length (TDL) increase at a concentration of 10 µM (fig. 4A, n=4 independent experiments (4 different cell cultures)), while it had no effect on the number of dendritic endpoints (EP) (fig. 4B). The Kruskall-Wallis test was also performed in this phase, in order to compare non-parametric data between more than two groups: *p <0.05, **p<0.01, ***p<0.001.

In a seventh phase, the MeCP2-KO neurons were treated from DIV 3 to DIV 6 with the 14 drugs + mirtazapine administered in pairs, obtaining 105 pairs that were tested at three different concentrations (10 µM, 1 µM and 0.1 µM), for a total of 315 combinations. To quantify the effects of the interactions, the same morphological parameters used to test the individual drugs at DIV 6 were measured, namely total dendritic length (TDL) and the number of terminal branches (EP = Endpoints).

Of these 315 combinations, some pairs were more promising, since they produced positive effects at a concentration lower than that used for treatment with single drugs. This is important because lower drug concentrations are better tolerated by patients since they have fewer side effects.

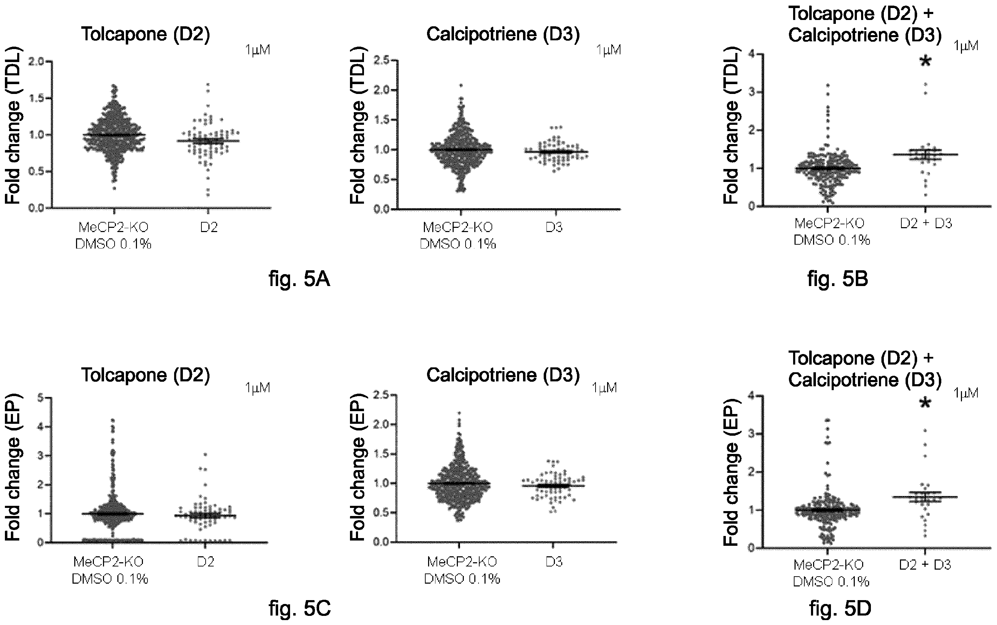

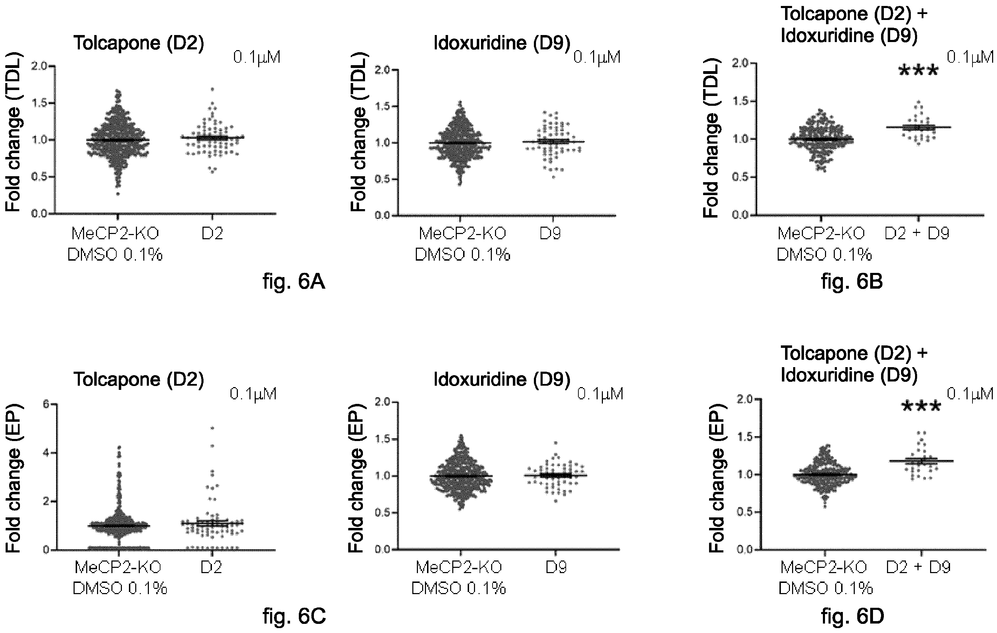

The combinations of the drugs tolcapone (identified with D2) and idoxuridine (D9) at a concentration of 0.1 µM, and the combination of tolcapone (D2) with calcipotriene (D3) at a concentration of 1 µM were able to counteract neuronal atrophy in MeCP2-KO mouse neurons, a model of Rett syndrome, at a concentration that is lower than the minimum effective concentration in the in vitro assays carried out with each of the individual drugs that had been 10 µM, thus indicating an enhancement of the effect found with the pair of drugs, compared to each of the two drugs used individually. Two other pairs, the tolcapone (D2) and melengestrol acetate (D12) pair, and the tolcapone (D2) and rolipram (D7) pair, had an effect on both the TDL as well as the EP, but at a concentration of 10 µM, and therefore did not produce a significant enhancement compared to the individually tested drugs (Table 1, fig. 5, fig. 6).

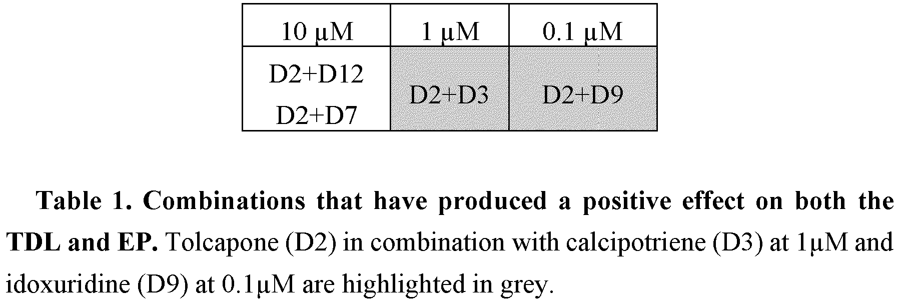

Figs. 5A-5D show the effect of tolcapone and calcipotriene, individually or in combination, on the total dendritic length (TDL) or the terminal branches (EP). In these experiments, tolcapone and calcipotriene are both at the concentration of 1µM, both when included individually or in combination. The results show that tolcapone and calcipotriene administered individually at 1µM had no positive effect on either the TDL (fig. 5A) or the terminal branches (fig. 5C). Tolcapone in combination with calcipotriene at 1 µM had a positive effect on both the TDL (fig. 5B) and also the terminal branches (fig. 5D).

Figs. 6A-6D show the results of the same tests, but performed on tolcapone and idoxuridine at 0.1 µM. Tolcapone and idoxuridine administered individually at 0.1 µM have no positive effect on either the TDL (fig. 6) or the terminal branches (fig. 6C). Tolcapone in combination with idoxuridine at 0.1 µM had a positive effect on both the TDL (fig. 6B) and also the terminal branches (fig. 6D).

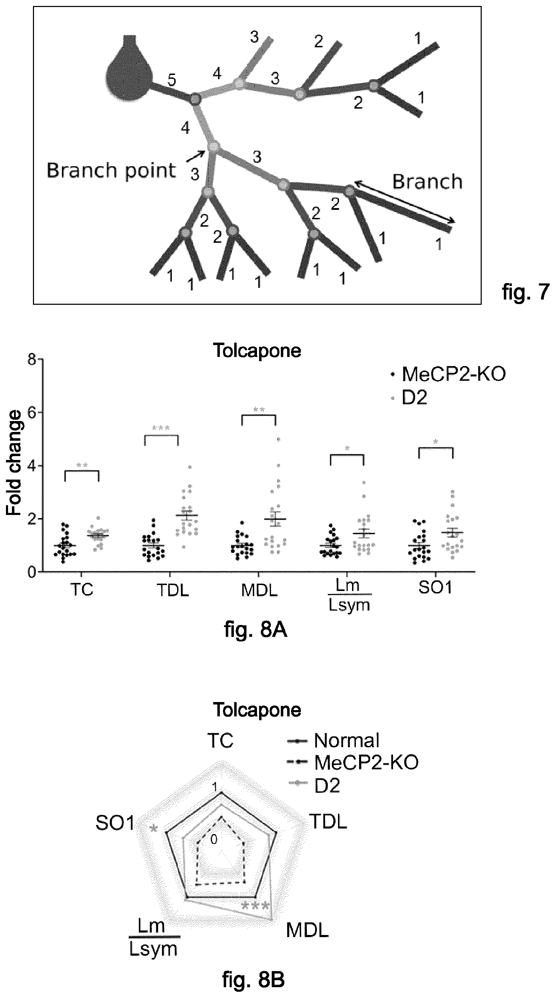

In an eighth phase, the drug tolcapone was tested to verify its efficacy in counteracting neuronal atrophy in more mature neurons. In this series of experiments, MeCP2-KO mouse hippocampal neurons were incubated with the drug for 3 days from DIV 9 to DIV 12. To visualize the dendrites and cell soma, the neurons were transfected with a plasmid encoding the Green-Fluorescent protein (GFP) that is expressed throughout the cytoplasm of neurons. Subsequently, the neurons were fixed, and their images were acquired with a fluorescence microscope to then be manually traced with the software NeuronJ (NIH-ImageJ), and then subjected to Sholl and Strahler analysis and measurement of the lengths of all dendritic processes. There were 5 measurements carried out:
1) total dendritic length (TDL), which gives an indication of the overall growth of the neurons;
2) Sholl analysis, which provides an estimate of the branches of the dendrites, both as a total number (TC = Total Crossings) and also as the number of branches at progressive distances from the neuron's cell body;
3) Strahler analysis, which counts the number of the different orders of stages of the dendrites. Strahler order 1 (SO1) stages are the outermost and most peripheral (= the tips of the dendritic tree), while order 5 (SOS) stages are the innermost and closest to the cell body (= the trunks at the base of the dendritic tree) (fig. 7);
4) maximal dendritic length (MDL), which gives information on the extension of the dendritic tree;
5) polarization index (PI Lm/Lsym), which is a measure closely related to the previous one (MDL) and indicates how close a neuron is to the elongated shape, compared to the symmetrical shape.

In order to achieve a complete recovery of the deficits, not only do Rett neurons have to reach a dendritic length comparable to healthy neurons, but they also have to have a harmonic breakdown between main and secondary stages, with a number of overall branches as close as possible to normal neurons, and an overall shape of the dendritic tree adequate for the neuron's function.

The results following treatment of MeCP2-KO neurons, model of Rett syndrome, with 10µM tolcapone (D2) are shown in fig. 8.

Fig. 8A shows the graph comparing the effect of tolcapone (in grey) compared to the effect of the carrier DMSO 0.1% (in black) on MeCP2-KO neurons. There is an increase in TC, TDL, MDL, Lm/Lsym and SO1 parameters with tolcapone compared to the treatment with DMSO.

Fig. 8B shows, in the center of each radial diagram with a dashed black line, the values of the 5 parameters for the MeCP2-KO neurons treated with the carrier (between 0 and 0.5). Externally, solid black line, the values (=1) for normal neurons (WT) are shown. The corresponding values after treatment with tolcapone (D2) are indicated in grey. A complete recovery of the values for the polarization index (Lm/Lsym) and the total dendritic length (TDL) parameters is observed and, while with regard to the total number of crossings in the Sholl analysis (Total crossings = TC) and the number of dendritic endpoints (Strahler Order 1 = SO1), a recovery that is partial and does not reach the normal values of the WT is observed, while the maximum dendritic length (MDL), that is, the length of the longest dendrite, is greater than normal.

It is clear that modifications and/or additions of parts may be made to the composition as described heretofore, without thereby departing from the field and scope of the present invention, as defined by the claims.

It is also clear that, although the present invention has been described with reference to some specific examples, a person of skill in the art will be able to achieve other equivalent forms of composition for use in the treatment of Rett syndrome, having the characteristics as set forth in the claims and hence all coming within the field of protection defined thereby.

In the following claims, the sole purpose of the references in brackets is to facilitate their reading and they must not be considered as restrictive factors with regard to the field of protection defined by the claims.

## Claims

1. Tolcapone for use in the treatment of Rett syndrome.

2. Combination of tolcapone and at least one other drug chosen from calcipotriene, idoxuridine, melengestrol acetate and rolipram for the treatment of Rett syndrome.

3. Composition for use in the treatment of Rett syndrome, **characterized in that** it comprises tolcapone.

4. Composition for use as in claim 3, **characterized in that** tolcapone acts as a promoter of neuronal maturation.

5. Composition for use as in claim 3 or 4, **characterized in that** it comprises, in addition to tolcapone, at least one other drug chosen from calcipotriene, idoxuridine, melengestrol acetate and rolipram.

6. Composition for use as in claim 5, **characterized in that** the concentration ratio between tolcapone and said at least one other drug is equal to 1:1.

7. Composition for use as in claim 5 or 6, **characterized in that** it comprises tolcapone and calcipotriene.

8. Composition for use as in claim 7, **characterized in that** the concentration of each of tolcapone and calcipotriene is between 0.2 µM and 5 µM.

9. Composition for use as in claim 5 or 6, **characterized in that** it comprises the drugs tolcapone and idoxuridine.

10. Composition for use as in claim 9, **characterized in that** the concentration of each of tolcapone and idoxuridine is between 0.02 µM and 0.5 µM.
